# EUROPEAN PATENT SPECIFICATION

(11) **EP 0 927 165 B1**
(45) Date of publication and mention of the grant of the patent: **19.11.2003**
(21) Application number: 97941402.6
(22) Date of filing: 04.09.1997
(51) Int. Cl.: C07D 217/00

(54) **INTERMEDIATES FOR MAKING HIV-PROTEASE INHIBITORS AND METHODS OF MAKING HIV-PROTEASE INHIBITORS**
ZWISCHENPRODUKTE ZUR HERSTELLUNG VON HIV-PROTEASE-INHIBITOREN UND VERFHAREN ZUR HERSTELLUNG HIV-PROTEASE-INHIBITOREN
INTERMEDIAIRES PERMETTANT LA PRODUCTION D'INHIBITEURS DE LA VIH-PROTEASE ET PROCEDES DE PRODUCTION D'INHIBITEURS DE LA VIH-PROTEASE

(30) Priority: 05.09.1996 US 708411
(43) Date of publication of application: 07.07.1999
(60) Divisional application: 03013275.7
(73) Proprietor: AGOURON PHARMACEUTICALS, INC., La Jolla, CA 93057-1022 (US); JAPAN TOBACCO INC., Takatsuki, Osaka 569 (JP)
(72) Inventor: WHITTEN, Kathleen, R., San Diego, CA 92130 (US); DEASON, Michael, E., Poway, CA 92064 (US)
(74) Representative: Grünecker, August, Dipl.-Ing.
(86) International application number: US9715468
(87) International publication number: WO98009951

(56) References cited:
- EP-A- 0 579 223
- WO-A-96/28423
- WO-A-97/11937
- WO-A-97/30993
- US-A- 5 484 926
- PELTIER ET AL: "Contribution à l'étude du group carboxylique. Absorption infrarouge et ionisation." BULL.SOC.CHIM.FR., 1960, pages 1141-1147, XP002049745
- DATABASE XFIRE beilstein 3-methoxy-2-methyl-benzoic acid ethyl ester, XP002049746 & MUKHARJI: INDIAN J.CHEM., 1971, pages 515-523,

## Description

Treatment of HIV-infected individuals is one of the most pressing biomedical problems of recent times. A promising new therapy has emerged as an important method for preventing or inhibiting the rapid proliferation of the virus in human tissue. HIV-protease inhibitors block a key enzymatic pathway in the virus resulting in substantially decreased viral loads, which slows the steady decay of the immune system and its resulting deleterious effects on human health. The HIV-protease inhibitor nelfinavir mesylate of formula 7 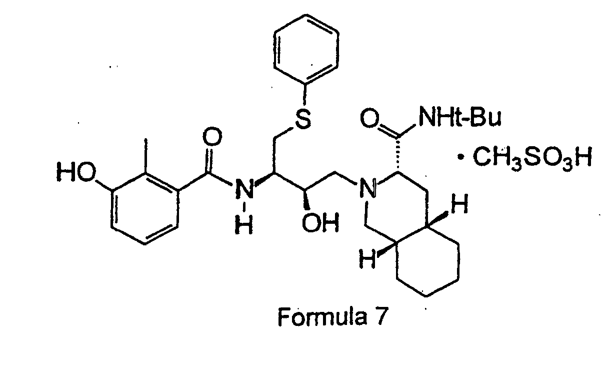 has been shown to be an effective treatment for HIV-infected individuals. Nelfinavir mesylate is disclosed in US-A-5,484,926.

The present inventors have discovered useful methods of making intermediate compounds that can be used in several reaction schemes to make nelfinavir mesylate. The present inventors also have discovered new methods for making nelfinavir mesylate from the free base nelfinavir of formula 4: 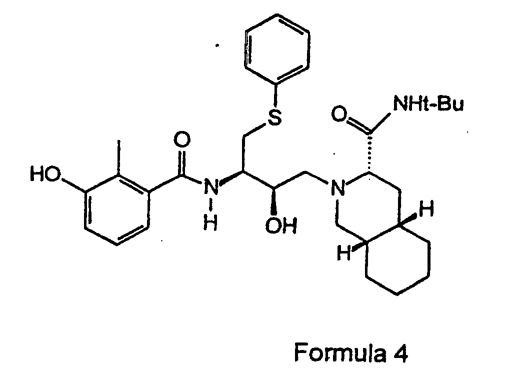 The nelfinavir free base also is disclosed in US-A-5,484,926.

It is an object of this invention to provide methods of making compounds and intermediates useful for making HIV-protease inhibitors and methods of making HIV-protease inhibitors. Such inhibitors are useful for treating HIV-infected individuals.

The present invention provides methods as defined in the claims:
(1) a method of making a compound of formula 3 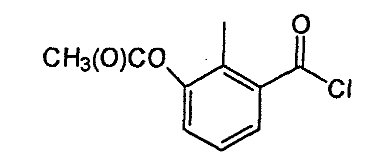 comprising adding an acetyl group and a leaving group being chlorine to a compound of formula 1 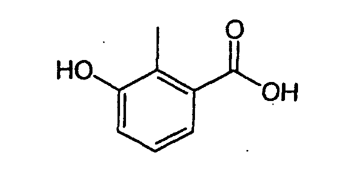 to form the compound of formula 3;
(2) a method of making a compound of formula 3 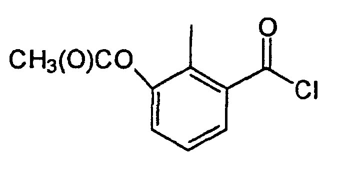 comprising adding a leaving group being chlorine to a compound of formula 2 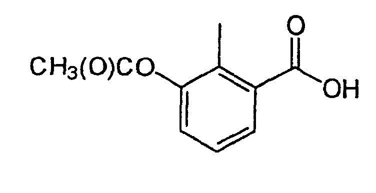 to form the compound of formula 3;
(3) a method of making a compound of formula 4 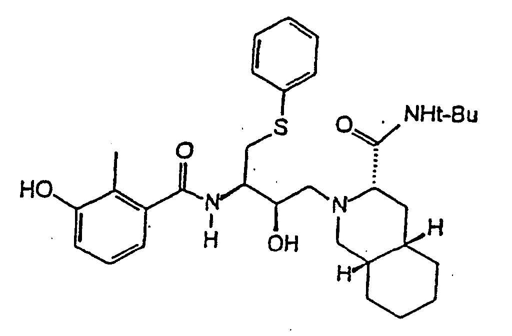 comprising deprotecting a compound of formula 5 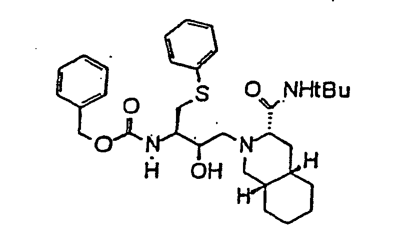 by treatment with an aqueous or alcoholic solution of an inorganic base, an aqueous or alcoholic solution of an acid, a solution of a protic organic acid and an inorganic acid, or by hydrogenolysis with a palladium or platinum catalyst; and
   adding the deprotected compound of formula 5 to a compound of formula 3 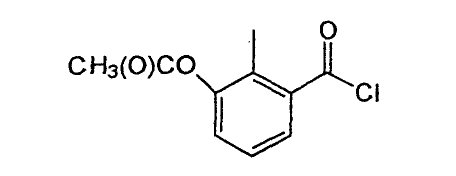 in an organic solvent to form the compound of formula 4;
(4) a method of making a compound of formula 4 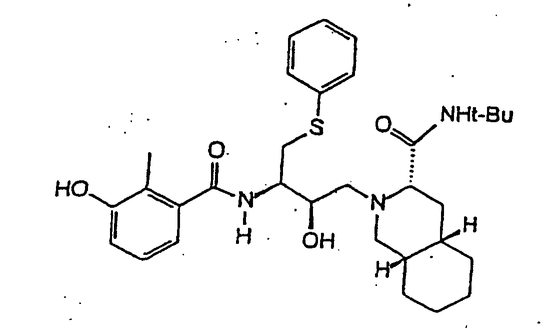 comprising combining a compound of formula 3 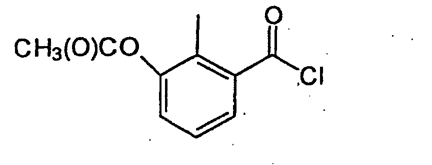 in an organic solvent with a compound of formula 6 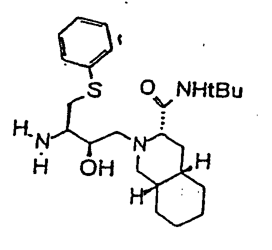 to obtain the compound of formula 4;
(5) a method of making a compound of formula 7 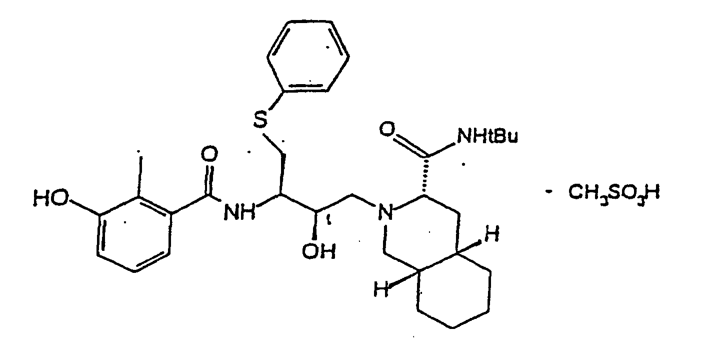 comprising deprotecting a compound of formula 5 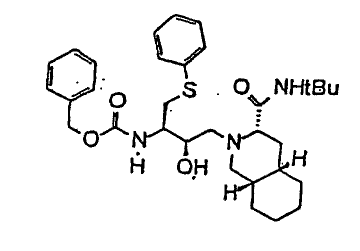 by treatment with an aqueous or alcoholic solution of an inorganic base, an aqueous or alcoholic solution of an acid, a solution of a protic organic acid and an inorganic acid, or by hydrogenolysis with a palladium or platinum catalyst; and adding the deprotected compound of formula 5 to a compound of formula 3 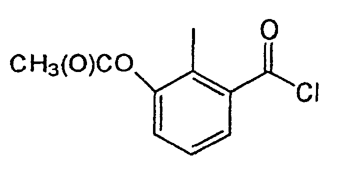 in an organic solvent to form a compound of formula 4 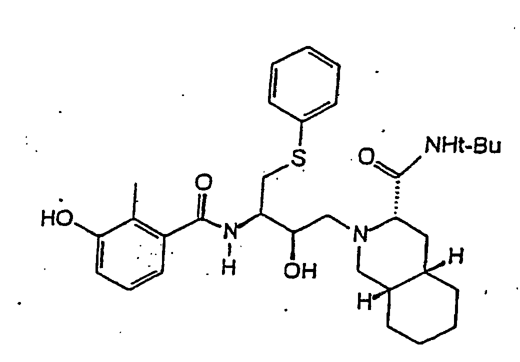 and converting the compound of formula 4 to the compound of formula 7 by treatment with methanesulfonic acid in an organic solvent; and
(6) a method of making a compound of formula 7 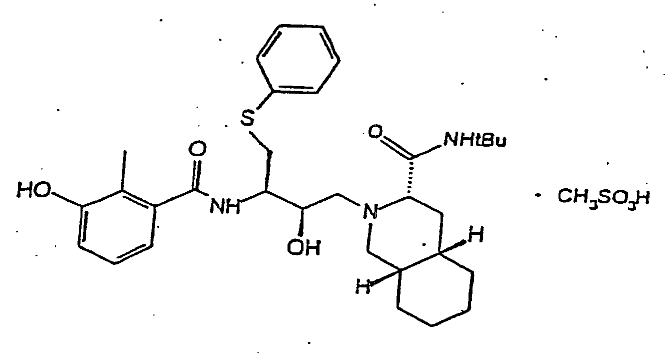 comprising combining a compound of formula 3 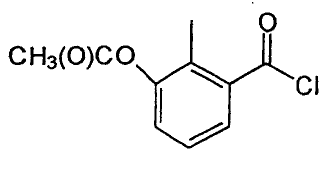 in an organic solvent with a compound of formula 6 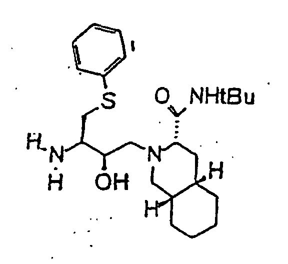 to form a compound of formula 4 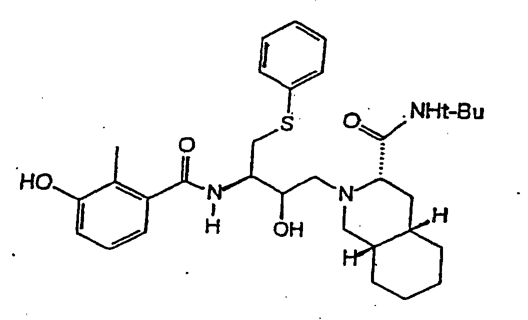 and converting the compound of formula 4 to the compound of formula 7 by treatment with methanesulfonic acid in an organic solvent. The conversion of the compound of formula 4 to the compound of formula 7 takes place by:
   (a) contacting the compound of formula 4 with an organic solvent;
   (b) contacting the compound of formula 4 with methanesulfonic acid to form a compound of formula 7; and
   (c) spray drying the compound of formula 7. in a more specific embodiment of this method, the organic solvent is ethanol.

In another method for making a compound of formula 7 from a compound of formula 4, the following procedure is followed:
(a) the compound of formula 4, a suitable solvent, and methanesulfonic acid are combined to form the compound of formula 7, the compound of formula 7 being dissolved in solution;
(b) a first antisolvent is added to the solution containing the compound of formula 7;
(c) the compound of formula 7 and the first antisolvent are agitated together to form a product having a solid phase and a liquid phase; and
(d) the product is filtered and washed with a second antisolvent, the second antisolvent being the same as or different from the first antisolvent, to obtain a solid final product according to formula 7. After the solid final product is washed, it can be dried by any appropriate method or means. Tetrahydrofuran can be used as the solvent, and diethylether can be used as at least one antisolvent, preferably at least the first antisolvent.

This invention relates to methods of making compounds and intermediates useful for making HIV-protease inhibitors and methods of making HIV-protease inhibitors.

The present invention is directed to various methods of making compounds of formulae 3, 4 (nelfinavir free base), and 7 (nelfinavir mesylate), as described above. Other methods of using compounds of Formulae 2 and 3 are disclosed in JP 95-248183 and JP 95-248184.

The term "DABCO" as used herein refers to the reagent 1,4-diazabicyclo[2.2.2]octane.

The term "DBN" as used herein refers to the reagent 1,5-diazabicyclo[4.3.0]non-5-ene.

The term "DBU" as used herein refers to the reagent 1,8-diazabicyclo[5.4.0]undec-7-ene.

The term "DMF" as used herein refers to the solvent N,N-dimethylformamide.

The term "NMP" as used herein refers to the solvent N-methyl-2-pyrolidinone.

The term "THF" as used herein refers to the solvent tetrahydrofuran.

The term "hydrogenolysis" as used herein refers to a reaction in which a single bond is broken and hydrogens become bonded to the atoms that were formerly bonded.

Compounds that contain at least one chiral center can exist as single stereoisomers, racemates, and/or mixtures of enantiomers and/or diastereomers. Preferably, the compounds are used in a form that contains at least 90% of a single isomer (80% enantiomeric or diastereomeric excess), more preferably at least 95% (90% e.e. or d.e.), even more preferably at least 97.5% (95% e.e. or d.e.), and most preferably at least 99% (98% e.e. or d.e.). Compounds identified herein as single stereoisomers are meant to describe compounds used in a form that contains at least 90% of a single isomer.

The compound 3 can be prepared by the novel methods of the present invention, which are described in detail below. Additionally, compound 3 can be used to prepare nelfinavir free base and nelfinavir mesylate according to the inventive methods described below.

A reaction scheme for the conversion of 3-hydroxy-2-methylbenzoic acid to nelfinavir free base is as follows: 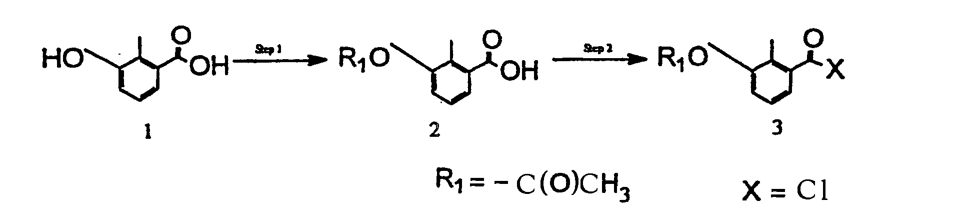 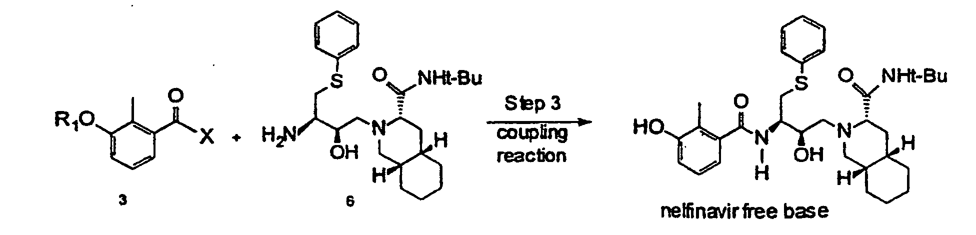 The acid **1** is commercially available from Lancaster Labs and Sugai Chemical Industries, Ltd. in Japan. The acid **1** also can be obtained according to the procedure described in US-A-5,484,926, for the Preparation of 9C.

R₁ can be installed onto 3-hydroxy-2-methylbenzoic acid (Step 1) using the corresponding acyl halide or anhydride in typical organic solvents for these types of reactions, such as halogenated solvents, ethers, and hydrocarbons accompanied by a base. Such bases typically are inorganic bases, such as metal hydroxides, bicarbonates, and carbonates, or organic bases, such as amines like triethylamine, diethylamine, diethyl isopropylamine, DABCO, or related di- or trialkylamines, as well as amidine bases like DBU and DBN. These reactions typically are run anywhere from below room temperature to approximately 100 °C. Alternatively, the esterification can be catalyzed by acids such as sulfuric acid when used in conjunction with an anhydride.

The group X (chlorine) is installed in Step 2 by reaction of the carboxylic acid derivative **2.** The acyl halides of formula **3** can be prepared using inorganic halogenating agents such as thionyl chloride, phosphorus trichloride, phosphorus pentachloride, or organic agents such as oxalyl chloride or trichlorisocyanuric acid. This process can be catalyzed by DMF or a related alkyl amide.

The coupling of compound **3** to amine **6** (Step 3) can be carried out in a variety of ways. The coupling can be performed in most common organic solvents such as THF; diethyl ether, dioxane, methyl, t-butyl ether, or other ethers; acetone, cyclohexanone, methyl isobutylketone and other ketones; esters such as ethyl, methyl, and isopropyl acetate; halogenated solvents such as halogenated methanes and ethanes; chlorobenzene and other halogenated benzenes; nitriles such acetonitrile and propionitrile; lower alcohols such as ethanol, isopropanol, t-butanol; and related alcohols; and polar organic solvents such as dimethylformamide, dimethylsulfoxide, N-methyl-2-pyrolidinone, and related amide-containing solvents. A base frequently is used and can be any of a number of inorganic bases (such as metal hydroxides, bicarbonates, and carbonates) or organic bases (such as amines like triethylamine, diethylamine, diethyl isopropylamine, DABCO, or related di- or trialkylamines, as well as amidine bases like DBU and DBN).

Protecting group removal is accomplished using any of the standard methods for deprotecting a particular class of protecting group. Esters and carbonates usually are removed with aqueous or alcoholic solutions of inorganic bases, such as metal hydroxides, carbonates, and bicarbonates, at ambient temperatures up to 100 °C. Ethers are deprotected using boron-based Lewis acidic compounds such as BBr₃ and BCl₃, alkyl thiolates, or trialkylsilyl halides. Either ether or carbonate protecting groups that contain benzyl groups bonded to heteroatoms can be removed by hydrogenolysis with a palladium or platinum catalyst. Acetal-based protecting groups can be removed under aqueous or alcoholic acidic conditions, promoted by Lewis acids such as transition metal halides or halides of the Group 3 metals, or by protic organic acids (such as p-toluenesulfonic and related alkyl and arylsulfonic acids, trifluoroacetic acid and related organic carboxylic acids with a pK of less than 2) and inorganic acids (such as sulfuric, hydrochloric, phosphoric, and nitric acids). Silylether protecting group removal can be accomplished by aqueous or alcoholic acid or base or by fluoride ion promoted desilylation by use of inorganic fluoride sources such as potassium or cesium fluoride or by tetralkylammonium fluoride salts.

Nelfinavir mesylate can be prepared from 3-acetoxy-2-methylbenzoyl chloride (acid chloride). The acid chloride can be prepared from the corresponding 3-hydroxy-2-methylbenzoic acid in the following two step procedure: 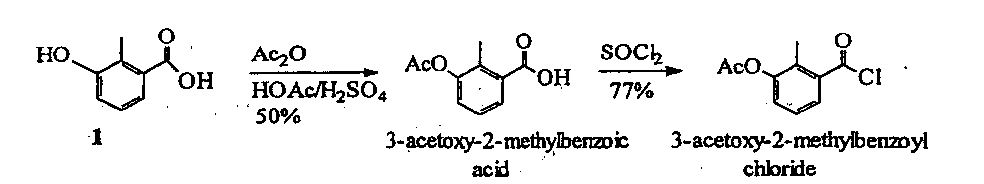 In the production of the acid chloride, the acid **1** is converted to the acetoxy acid (a compound of formula **2**), which is treated with thionyl chloride to give 3-acetoxy-2-methylbenzoyl chloride in good yield.
The acid chloride then can be coupled to the amine **6** under classical conditions resulting in the production of nelfinavir free base as follows: 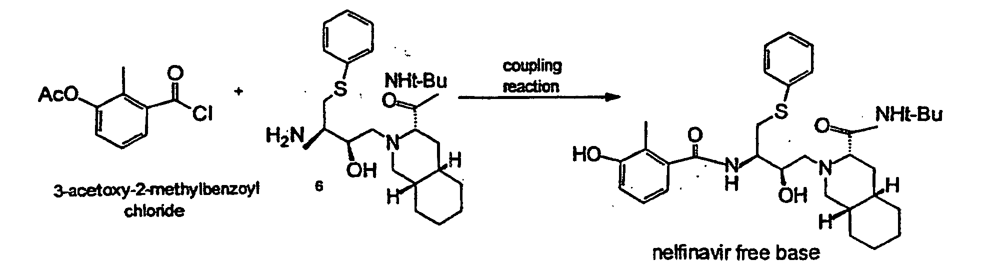 The acid chloride is treated with the amine **6** in the presence of triethylamine in THE at ambient temperature for 30 minutes followed by an aqueous basic hydrolysis of the acetate group to give nelfinavir free base. The free base can be converted to nelfinavir mesylate by methods described in more detail below.

### Preparation of Nelfinavir Free Base from 3-Acetoxy-2-Methylbenzoic Chloride

### Summary of the Process

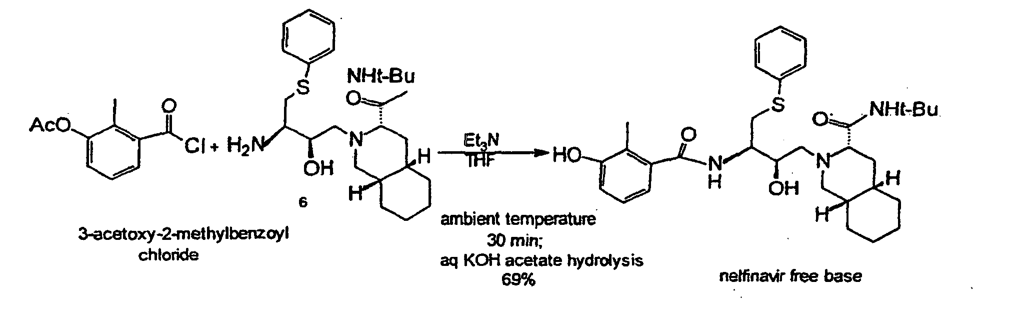

To obtain 3-acetoxy-2-methylbenzoyl chloride, 3-hydroxy-2-methylbenzoic acid was slurried in acetic acid with acetic anhydride and catalytic sulfuric acid. Acetylation of the hydroxy group was complete within two hours at ambient temperature. After complete reaction, the resulting slurry was poured into water, and the product was isolated by filtration. The wet cake was reslurried in water, isolated by filtration, and dried under vacuum. Product was obtained in 80-90 % yield with an apparent purity of 89-92% by HPLC. Crude, dry 3-acetoxy-2-methytbenzoic acid was dissolved in four volumes of ethyl acetate with refluxing. The resulting solution was cooled to <70 °C, and five volumes of hexanes were added. The mixture was returned to reflux and then cooled to <10 °C for 1 hour. The slurry was filtered, rinsing the reactor with filtrate. The product was dried under vacuum. Recrystallization improved the HPLC UV apparent purity from 89 - 92% to >98%. The single largest impurity dropped from 4 - 5% to ∼0.5%. The product was 3-acetoxy-2-methylbenzoic acid.

3-Acetoxy-2-methylbenzoic acid was slurried in methyl-*t*-butyl ether (MTBE) and treated with 1.2 equivalents of thionyl chloride and catalytic dimethylformamide. After three hours at ambient temperatures, the reaction was complete, giving a brown solution. Solvent (MTBE) was removed by vacuum distillation. Residual thionyl chloride was removed by addition of toluene followed by vacuum distillation. The resulting 3-acetoxy-2-methylbenzoyl chloride was isolated either directly as an oil or by crystallization from two volumes of heptane at <10 °C. Product was obtained in >100% yield when isolated as an oil and 82 - 85% yield'when crystallized from heptane.

To obtain compound **6** for the coupling, a compound of formula **5** (made as described below) was refluxed in a mixture of ethanol and aqueous NaOH to cleave the CBZ protecting group forming a compound of formula **6.** Water and HCl were added to dissolve the Na₂CO₃ and neutralize excess NaOH, giving a biphasic mixture. The mixture was cooled, and the lower aqueous layer was removed. Triethylamine was added followed by a solution of 3-acetoxy-2-methylbenzoyl chloride in tetrahydrofuran to give an acetate of the compound of formula **4.** Aqueous NaOH was added, and the mixture was heated to reflux to give a compound of formula **4.** The mixture was concentrated at atmospheric pressure to remove tetrahydrofuran, triethylamine, and most of the ethanol. The mixture was added to a heated solution of water and glacial acetic acid to precipitate the product. The pH was adjusted with additional acid, and the solids were filtered off while hot. The wet cake was rinsed with hot water and dried to give crude nelfinavir free base.

This method is described in more detail below

### Preparation of 3-Acetoxy-2-methylbenzoic acid

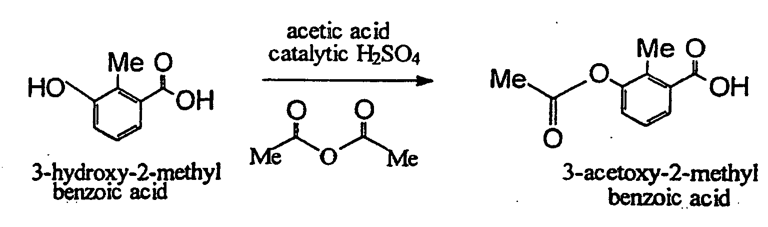

### PROCEDURE:

### Materials

| | | | | |
|---|---|---|---|---|
| 3-hydroxy-2-methylbenzoic acid | FW 152.15 | | 3500 g | 1.0 equiv |
| acetic acid | | | 8750 mL | |
| sulfuric acid | | | 70 mL | |
| acetic anhydride | FW 102.1 | d 1.082 | 2390 mL | 1.1 equiv |
| purified water | | | 28000 mL | |

Acetic acid (8750 mL), 3-hydroxy-2-methylbenzoic acid (3500 g), and sulfuric acid (70 mL) were charged into a 22 liter reactor. The reactor contents were stirred to give a homogeneous mixture. The mixture exothermed to 36 °C. Acetic anhydride (2390 mL) was added to the mixture in the 22 L reactor. An exotherm warmed the reactor contents from 36 to 44 °C. The reaction mixture was stirred at ambient temperature for two hours (reactor contents allowed to cool slowly). The reaction was tested for complete conversion of the starting material by TLC. The reaction mixture was generally a tan slurry at the completion of the reaction.

Purified water (17500 mL) was added to a 50 L extractor, and the reaction mixture from the 22 L reactor was added to this water. The 22 L reactor was rinsed into the 50 L extractor with purified water (3500 mL). The reaction mixture was vacuum filtered, washing the reactor and filter cake with purified water (3500 mL). The wet filter cake was transferred to a 50 L extractor, and purified water (14000 mL) was added, with stirring, to obtain a homogeneous slurry. The reslurried mixture was vacuum filtered, and the reactor and filter cake were rinsed with purified water (3500 mL). The filter cake was pulled as dry as possible and then transferred to drying pans. The product was dried in a vacuum oven at 60 - 80 °C and ≥28 mm Hg for 12 - 72 hours. Theoretical yield: 4466 g. Actual weight produced: 3910 g (87.6%). HPLC assay: 89.4% or 87.7%.

Purification was achieved as follows. The crude 3-acetoxy-2-methylbenzoic acid (3910 g from above) and ethyl acetate (16.0 L) were charged to a 50 L reactor. The reactor contents were heated to reflux (77 °C) until all solids went into solution. The reactor contents were cooled to < 70 °C. Hexanes (19.5 L) were added to the reactor. The reactor contents were again heated to reflux (69 °C), and then the mixture was cooled to <10 °C for 1 hour. The cooled slurry from this step was vacuum filtered, and the reactor was rinsed with cold mother liquors. The filter cake was pulled as dry as possible and then transferred to drying pans. The product was dried in a vacuum oven at 60 - 70 °C and ≥28 mm Hg for 12 - 72 hours. Theoretical yield: 3910 g. Actual weight produced: 3128 g (80%). This procedure improves the HPLC UV apparent purity from 89 - 92% to >98%. The single largest impurity drops from 4 - 6% to <1%. The isolated product is a tan solid. ¹H NMR δ 8.0 (d, 1H), 7.3 (overlapping m, 2H), 2.5 (s, 3H), 2.3 (s, 3H).

### Preparation of 3-Acetoxy-2-methylbenzoyl Chloride

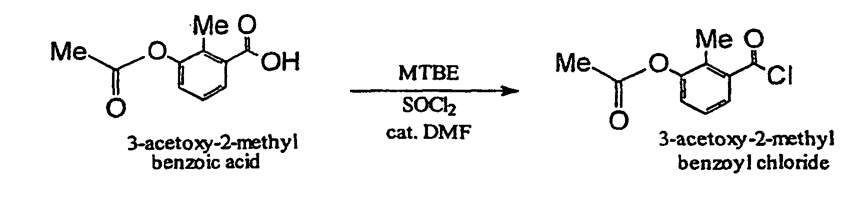

### PROCEDURE:

| Materials | MW | d | wt | equiv. |
|---|---|---|---|---|
| 3-acetoxy-2-methylbenzoic acid | 194.19 | | 3000 g | 1.0 |
| methyl-*t*-butyl ether | | | 12000 ml | |
| thionyl chloride | 118.97 | 1.638 | 1350 ml | 12 |
| dimethylformamide | 73.09 | 0.944 | 60 ml | 0.05 |
| toluene | | | 7500 ml | |
| heptane | | | 7500 ml | |

A 22 L reactor was purged with nitrogen and charged with recrystallized 3-acetoxy-2-methylbenzoic acid (3000 g), MTBE (12000 ml), and dimethylformamide (60 ml). The reactor contents were stirred to give a homogeneous mixture. Thionyl chloride (1350 ml) was added to the reactor. This reaction mixture was stirred at ambient temperature for 19 hours. (Generally no more than 3 hours are required for complete reaction, but the mixture can be held longer for convenience). The reaction solution was transferred to a Büchi rotovap, and the reactor was rinsed with toluene (1500 ml). The solution was concentrated as far as possible, maintaining the bath temperature at 40 - 50 °C. Toluene (6000 ml) was added to this concentrated solution. The toluene was distilled by rotovap to drive off excess thionyl chloride. The concentrate was transferred back to the 22 L reactor, and the Büchi flask was rinsed with heptane (6000 ml). The heptane mixture was cooled to <5 °C under nitrogen. After holding the crystallization mixture at <5 °C for > 30 minutes, the mixture was filtered, and the filter cake was washed with chilled heptane (1500 ml, <5 °C). The filter cake was dried in a vacuum oven at 15 - 20 °C and ≥28 mm Hg for 24 hours, giving a tan, granular solid. Theoretical yield: 3285 g. Actual weight produced: 2704 g (82.3%). HPLC assay 97.51 %; ¹H NMR δ 8.1 (d, 1H), 7.4 (overlapping m, 2H), 2.4 (s, 6H).

### Conversion of 3-Acetoxy-2-methylbenzoyl Chloride and Compound 6 to Nelfinavir Free Base

### REACTION SCHEME:

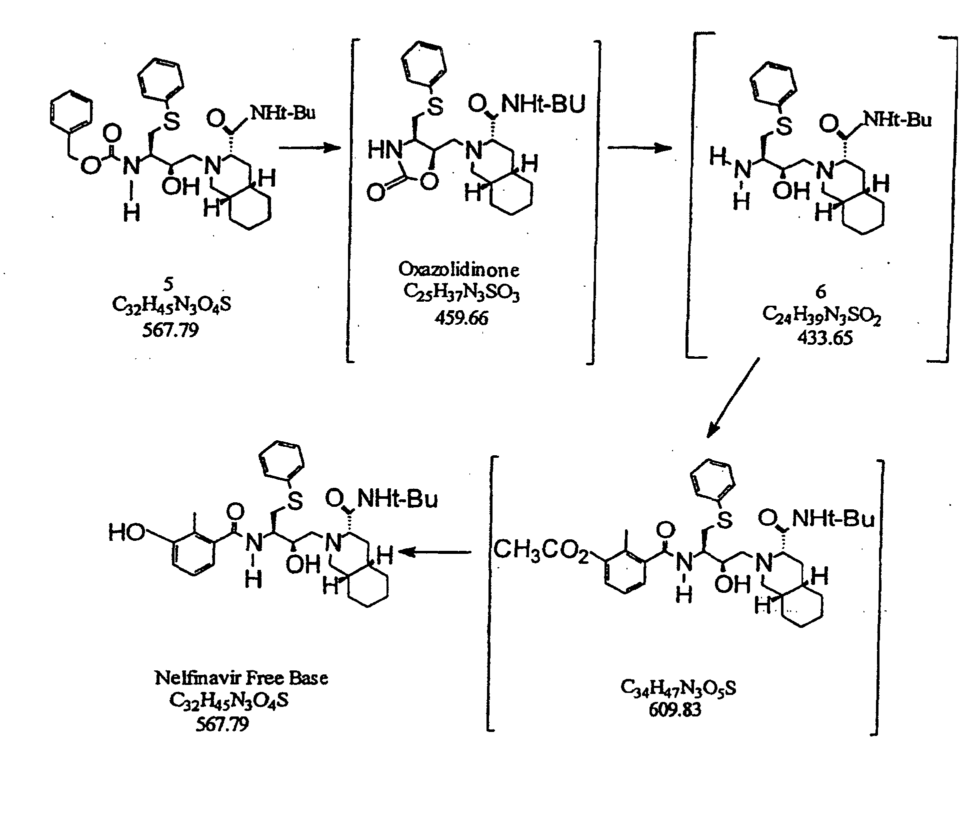

### Step A: Conversion of Compound 5 to Compound 6.

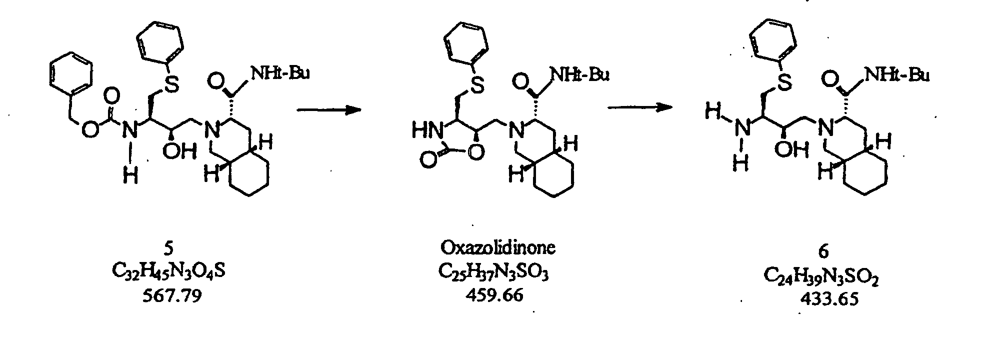

A 22 liter 3-neck round bottom flask ("RBF"), fitted with a condenser, temperature probe, and mechanical stirrer, was charged with a compound of formula **5** (1 kg, 97.7%, 1.72 mol, 1.00 eq) (which can be prepared as described below), ethanol (5 I, 95%), NaOH (280 ml, 50%, 5.33 mol, 3.1 eq), and water (2 I, DI). The mixture was stirred and heated to reflux (80-82 °C). All solids dissolved at 50 °C to give a clear yellow solution. The mixture became hazy with Na₂CO₃ precipitation as the reaction proceeded (Vol = 8280 ml). The deprotection was monitored by HPLC. Analysis at 210 minutes showed complete consumption of the compound of formula **5,** 0.95% oxazolidinone, 36% benzyl alcohol, and 62.5% of a compound of formula **6.** Analysis at 300 minutes showed 0.34% oxazolidinone, 36% benzyl alcohol and 62.6% of compound of formula **6.** Water (1260 ml) was added to the mixture to dissolve all solids, and the mixture was cooled to 67 °C (Vol = 9540 ml). HCl (344 ml, 6 N, 2.06 mol, 1.2 eq.) was then added to the mixture. The mixture was agitated 10 minutes and then allowed to settle for 20 minutes to give two layers (Vol = 9884 ml). The lower aqueous Na₂CO₃ layer was removed at 60 °C. The volume of the aqueous cut was 365 ml, pH = 14. The pH of the clear yellow upper layer was 10-10.5. The upper layer was used directly in the next step.

### Step B: Conversion of Compound 6 to an Acetate of Compound 4.

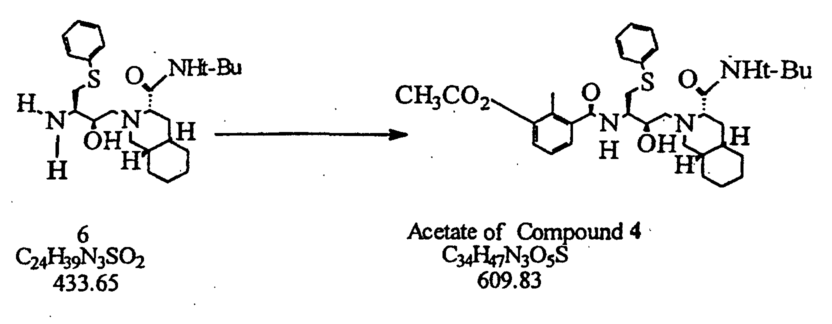

| **Chemicals** | | | | | | | | |
|---|---|---|---|---|---|---|---|---|
| | **source** | **assay** | **Kg** | **L** | **d** | **mw** | **mol** | **equiv** |
| Compound 6/EtOH | 1040-090 | | (0.746) | | | 433.65 | (1.72) | 1.00 |
| triethylamine | Fisher | | 0.26 | 0.36 | 0.726 | 101.19 | 2.58 | 1.50 |
| tetrahydrofuran | Fisher | | 0.40 | 0.45 | 0.889 | 72.11 | | |
| 3-acetoxy-2-methylbenzoylchloride | AC1322 | 98.9% | 0.39 | | | 212.63 | 1.81 | 1.05 |
| | | | | | | | | |
| Reference: | 1040-092 | | | | | | | |

The solution from Step A was cooled to 25 °C, triethylamine (360 ml, 2.58 mol, 1.50 eq) was added to the solution, and the mixture was cooled to 7 °C (pH = 11.5-12.0). The mixture became hazy at 23 °C (Vol = 9879 ml). This mixture was charged to a mixture of 3-acetoxy-2-methylbenzoyl chloride (388.5 g, 98.8 %, 1.81 mol, 1.05 eq) and tetrahydrofuran (440 ml) over 5 minutes. THF (10 ml) was used to complete the transfer. A 7.4 °C exotherm was observed. The mixture at the end of the addition was milky white. (Vol = 10,717 ml). HPLC analysis after 30 minutes showed <0.2% of a compound of formula **6,** 77% of the acetate of the compound of formula **4,** 18.2% benzylalcohol, and no ester present. The milky mixture was used directly in the next step.

### Step C : Saponification of Compound 4.

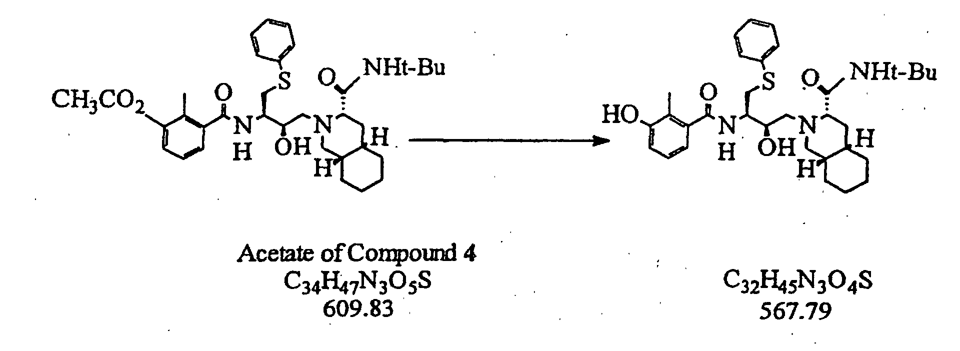

| **Chemicals** | | | | | | | | |
|---|---|---|---|---|---|---|---|---|
| | **source** | **assay** | **Kg** | **L** | **d** | **mw** | **mol** | **equiv** |
| Acetate of | | | | | | | | |
| Compound 4/PA | 1040-092 | | (1.05) | | | 609.83 | (1.72) | 1.00 |
| NaOH | Fisher | 50% | 0.55 | 0.36 | 1.515 | 40.00 | 6.88 | 4.00 |
| Water | DI | | 15.0 | 15 | 1.000 | 18.02 | | |
| HOAc, glacial | Fisher | 17.4 N | 0.25 | 0.23 | 1.049 | 60.05 | 4.07 | 2.37 |
| Ethanol, (5% | | | | | | | | |
| methanol) | McCormick | 95% | 0.04 | 0.05 | 0.785 | 46.07 | | |

NaOH (50%, 364 ml, 6.88 mol, 4.0 eq) was added to the mixture from Step B. The milky mixture became clear, then hazy, light brown. The mixture was agitated at 20°C for 35 minutes. HPLC showed 15.9% benzylalcohol, 78.6% of compound **4,** and no acetate (Vol = 11,081 ml). The mixture was heated to reflux and partially concentrated by atmospheric distillation until the head temperature reached 82°C. The distillate volume was 4275 ml. The pH of the mixture was 14. The pot volume was measured (Vol = 6000 ml).

Water (5 L) and HOAc (100 mL) were charged to a 12 L 3-neck round bottom flask fitted with a temperature probe and mechanical stirrer. The solution was heated to 54 °C (pH = 2-2.5) (Vol = 5100 ml). One half of the compound 4 mixture produced above (3 L) was added to this warm aqueous acetic acid solution to precipitate fine white solids. The pH was then adjusted to 7-7.5 with HOAc (19 ml), and the temperature was 53 °C (Vol = 8119 ml). The solids were filtered off at 53 °C using isolated vacuum. The filtration was quick and easy. The reactor and wet cake were rinsed with warm (35 °C) water (2.5 L), and the filtrates were combined. The wet cake was pulled dry for 15-20 minutes.

Water (5 L) and HOAc (100 mL) were charged again to the 12 L 3-neck round bottom flask. The solution was heated to 41 °C (Vol = 5100 ml). The remaining half of the compound 4 reaction mixture (3 L) was added to the new warm aqueous acetic acid solution to precipitate fine white solids. The pH was then adjusted to 7-7.5 with HOAc (15 ml). The temperature was 44 °C (Vol = 8115 ml). The solids were filtered off at 53 °C using isolated vacuum. The filtration was quick and easy. The reactor and wet cake were rinsed with warm (35 °C) water (2.5 L), and the filtrates were combined. The wet cake was pulled dry for 15-20 minutes.

The two wet cakes (3587 g) were dried under vacuum at 60 °C for 90 hours to give a dry wt of 1075.38 g crude Compound **4.** Theoretical yield is 977 g.

### Step D : Purification of Compound 4

| **Chemicals** | | | | | | | | |
|---|---|---|---|---|---|---|---|---|
| | **source** | **assay** | **wt** | **ml** | **d** | **mw** | **mmol** | **equiv** |
| Crude Compound 4 | 895-131 | 91.82% | 290 g | | | 567.79 | 469 | 1.00 |
| Acetone | Fisher | | 4038 g | 5105 | 0.791 | 58.08 | | |
| Water | DI | | 1070 g | 1070 | 1.000 | 18.02 | | |
| Celite | Aldrich | | 29 g | | | | | |
| Darco G-60 activated | Fisher | | 44 g | | | 12.01 | | |
| carbon | | | | | | | | |

A 12 liter 3-neck RBF, fitted with a condenser, temperature probe, and mechanical stirrer, was charged with crude compound **4** (290 g, 92%, 469 mmol); activated carbon (Darco G-60, 44g), acetone (4305 ml), and water (870 ml, DI). The mixture was heated to reflux (60-64°C) and held 45 minutes (Vol = 5509 ml). The hot slurry was filtered through celite (29 g) using isolated vacuum. The reactor and filter cake were rinsed with acetone (200 ml), and the clear, light yellow filtrates were combined. The mixture was allowed to cool slowly to 25°C over 2.5 hours with stirring to precipitate a fine white solid (Vol = 5665 ml). The white slurry was cooled to 0-10 °C and held for 1 hour. The solids were filtered off using isolated vacuum, and the liquid level was pulled through the surface of the wet cake. The reactor and wet cake were rinsed with a cold (0-10 °C) mixture of acetone/water (2:1, 300 ml). The liquid level was pulled through the surface of the wet cake, and the reactor and wet cake were again rinsed with a cold (0-10 °C) mixture of acetone/water (2:1, 300 ml). The wet cake was pulled as dry as possible using isolated vacuum and rubber damming to give a wet weight of 581 g. The product was dried under vacuum at 65 °C for 16 hours to give a dry weight of 221.61 g of compound **4.** Theoretical yield was 266.28 g. HPLC and ROI analysis showed 99% and 0.14% respectively. Adjusted yield was 82%.

The present invention also is directed to novel methods of converting nelfinavir free base, compound **4,** to nelfinavir mesylate, compound **7.** These methods are described in more detail below, including the method for preparing compound **4** from compound **5** and the method for preparing compound **5.**

### Procedure for Preparation of Compound 5

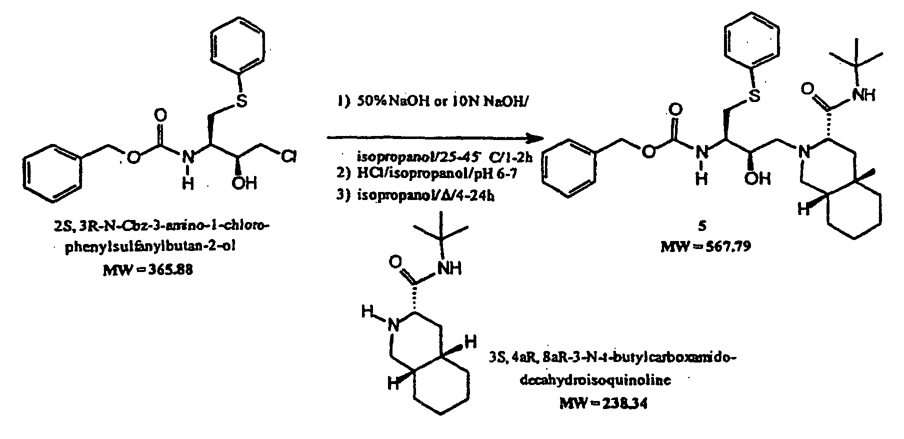

One equivalent 2S, 3R-N-Cbz-3-amino-1-chlorophenylsulfanylbutan-2-ol (which can be obtained from Kaneka Corporation or prepared as described in US-A-5,484,926) is stirred in a sufficient volume of methanol, ethanol, isopropanol, or other low boiling alcoholic solvent at 20° - 45°C. Isopropanol is the preferred solvent. A slight subcess of alkali base, such as sodium hydroxide or potassium hydroxide, as either an aqueous solution or as a solid, is added to this mixture with stirring. 10N sodium hydroxide is the preferred base. The mixture is stirred for 30 minutes to 24 hours until epoxide formation is complete. When the stir period is complete, the pH is adjusted to 6-7 with a proton acid such as HCl, either neat or dissolved in the reaction solvent.

A slight excess of 3S,4aR,8aR-3-N-t-butylcarboxamidodecahydroisoquinoline (which can be prepared as described in US-A-5,256,783) is added as either a dry solid or as a slurry to the reaction, and the mixture is heated to 40°C to reflux for 12-24 hours or until the reaction is judged to be complete. Alternatively, 3S,4aR,8aR-3-N-t-butylcarboxamidodecahydroisoquinoline can be introduced to the reaction at the same time that the 2S, 3R-N-Cbz-3-amino-1-chlorophenylsulfanylbutan-2-ol is charged to reactor. The epoxide formation is allowed to proceed as described. In this case, the reaction is not neutralized to a pH of 6-7, but a fixed amount of proton acid is added to neutralize excess base remaining. In either case, the reaction is partially concentrated *in vacuo*. The mixture is diluted with an equal volume of water and heated to reflux. Alternatively, the reaction is fully concentrated, and acetone or other ketonic solvent is added. The mixture can be filtered at this point, then an equal amount of water is added, and the mixture is heated. The resultant mixture is cooled with stirring. The resultant slurry is filtered, washed with aqueous solvent, and dried to yield compound **5.**

### Procedure for Preparation of Nelfinavir Free Base (Compound 4)

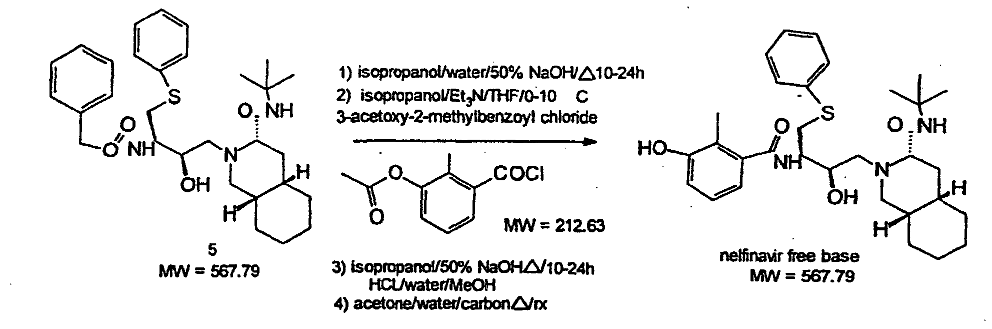

In addition to the procedure described above, the following procedure can be used to convert compound **5** to the nelfinavir free base (compound **4**):

One equivalent of compound **5,** an excess of alkali base (such as sodium hydroxide or potassium hydroxide), and an alcoholic solvent (such as methanol, ethanol, or isopropanol) are combined, and the mixture is heated at reflux with stirring. 50% caustic soda is the preferred base and isopropanol is the preferred solvent. Water can be added to facilitate solubility of the base. When the reaction is judged complete, the mixture is cooled to 30° to 35°C, and the lower aqueous layer, if any, can be removed. The mixture is cooled to below 25°C and an excess amount of organic base (such as diisopropylethyl amine or triethylamine) is added. Triethylamine is the base of choice.

A solution of excess 3-acetoxy-2-methylbenzoyl chloride in methanol, ethanol, isopropanol, THF, or other alcohol soluble solvents is slowly added to the cold mixture with stirring. THF is the preferred solvent.

An excess of alkali base, such as sodium hydroxide or potassium hydroxide, is added, and the mixture is heated at 40°C to reflux with stirring. 50% caustic soda is the preferred base. When the reaction is judged complete, the mixture is cooled, and the lower aqueous layer is removed.

The reaction mixture is partially concentrated *in vacuo*. If deemed necessary, the mixture can be diluted with an alcohol solvent to facilitate stirring. Methanol is the preferred solvent The mixture is added to aqueous acid to form a slurry. HCl is the preferred acid. The pH is adjusted to 7-8 with aqueous acid. The slurry is filtered and washed with water. The wet cake can be reslurried in water. The crude product is dried (partially or completely) or can be taken into the next step wet.

Either the dry or the crude, wet product is dissolved in aqueous acetone at reflux in the presence of activated carbon. The hot mixture is filtered, water is added, and the entire mixture is cooled with stirring to form a slurry. The slurry is filtered, washed with aqueous acetone, and dried to give nelfinavir free base.

Other methods for preparing nelfinavir free base are disclosed in US-A-5,484,926.

### Procedure for Spray Drying Nelfinavir Free Base to Obtain Nelfinavir Mesylate

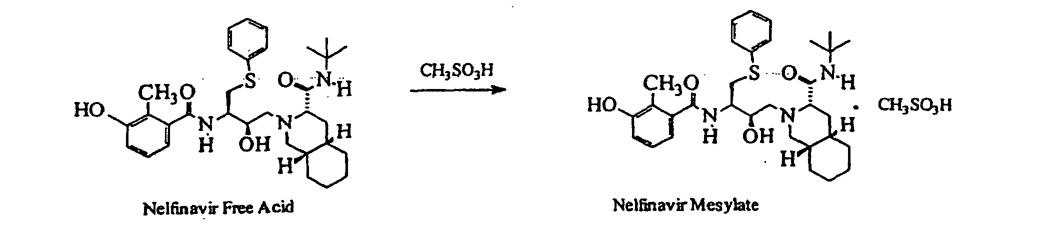

Generally, nelfinavir free base can be converted to nelfinavir mesylate using the following novel spray drying procedure.

Nelfinavir free base and an organic solvent (such as methanol, ethanol, isopropanol, THF, acetone, or MIBK) are mixed in a suitable vessel, and an equivalent amount of methanesulfonic acid is added. Ethanol is the preferred solvent. The mixture is stirred until nelfinavir mesylate is formed. The resultant slurry or solution is pumped into the spray dryer where the following settings are controlled:

| | |
|---|---|
| Inlet Temperature | 100-190°C |
| Outlet Temperature | 60-120°C |
| Atomizer Type | vane, cocurrent flow, or counter current flow |
| Drying Gas Rate | depends on equipment scale |

The inlet and outlet temperatures, feed rate, and atomizer type can be adjusted to optimize output and particle size distribution. Spray dried nelfinavir mesylate is collected at the spray dryer outlet collection point.

Specifically, this conversion was performed as described below.

19.4 kg ± 5 % Alcohol (USP, 190 proof) and 6.00 kg ± 1% nelfinavir free base were added to a clean, dry 20-40L stainless steel container. The mixture was stirred until homogenous, then 1.04 kg ± 1 % methanesulfonic acid, 99%, was added. The mixture was stirred until all solids were dissolved. A 0.2µ filter cartridge was connected to the pump inlet, and the alcohol solution was pumped through the filter into the spray dryer set with the following initial settings:

| | |
|---|---|
| Inlet Temperature | 160°C |
| Outlet Temperature | 90°C |
| Wheel Type | 50 mm vane wheel |
| Wheel Speed | 27000 rpm |
| Drying Gas Rate | 75 kgs./hour |

The inlet and outlet temperatures, feed rate, and wheel speed can be adjusted to optimize output and particle size distribution. The specific spray dryer used was a Niro Atomizer Portable Spray Dryer, type HT (equipped for inert gas) connected to an active carbon filter for removal of organic solvent residues. After the .bulk of the solution had been spray dried, the mixing tank was rinsed into the spray dryer with 1.0 kg ± 5% Alcohol, USP, 190 proof. The spray dried nelfinavir mesylate was collected in 80-100% theory yield.

### Procedure for Precipitation of Nelfinavir Free Base to Obtain Nelfinavir Mesylate

Alternatively, nelfinavir free base can be converted to nelfinavir mesylate using the following novel precipitation procedure.

Nelfinavir free base is slurried or dissolved in a suitable solvent (such as THF, methanol, or ethanol). THF is the preferred solvent. A molar equivalent amount of methanesulfonic acid is added, and the mixture is stirred until all solids dissolve. The solution is added to several volumes of an antisolvent (such as methyl t-butyl ether, diethyl ether, hexanes, or heptanes) that is rapidly stirring. Diethyl ether is the preferred antisolvent. After stirring, the mixture is filtered and washed with antisolvent. The solid is dried in a vacuum oven to yield nelfinavir mesylate.

Specifically, this conversion was performed as described below.

Nelfinavir free base (10.2 kg, 18.0 mol) and 24 L of tetrahydrofuran were added to a 100L reactor. Methanesulfonic acid (1.8 kg, 18.48 mol) also was added to the reactor. The reactor was stirred until all solids dissolved, and then the solution was filtered into a 100 gallon polypropylene tank containing 306 L methyl t-butyl ether or diethyl ether that was rapidly stirring. After stirring for 2 hours, the 100 gallon tank contents were filtered, washed with 17 L of methyl t-butyl ether or diethyl ether, and pulled as dry as possible. The solid was transferred to a rotocone drier and dried in a vacuum oven at 60-65°C (at least 26 in. Hg or higher vacuum) for 12-72 hours or until the methyl t-butyl ether or diethyl ether content of the dried solid was below 1%. If necessary, the drier contents could be milled in a Fitzmill grinder to accelerate drying. Typical yields of nelfinavir mesylate range from 9 to 11 kg. (76% - 92% theory).

## Claims

1. A method of making a compound of formula 3 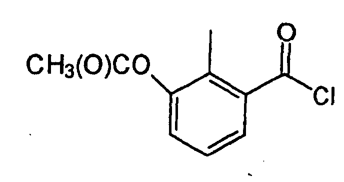 comprising adding an acetyl group and a leaving group being chlorine to a compound of formula 1 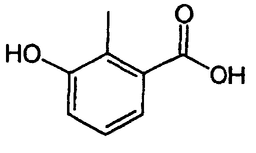 to form the compound of formula 3.

2. A method of making a compound of formula 3 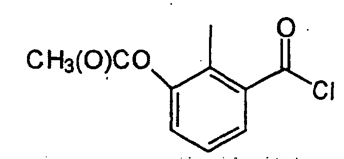 comprising adding a leaving group being chlorine to a compound of formula 2 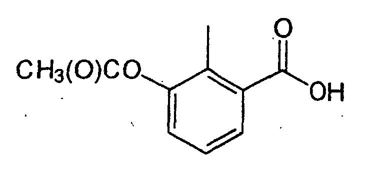 to form the compound of formula 3.

3. A method of making a compound of formula 4 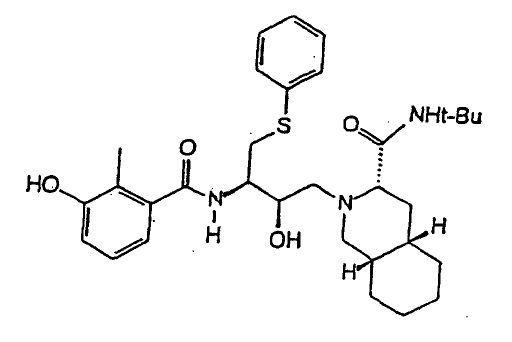 comprising deprotecting a compound of formula 5 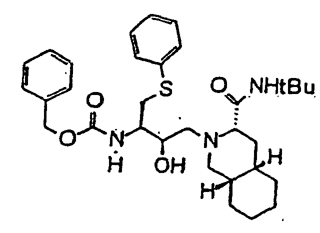 by treatment with an aqueous or alcoholic solution of an inorganic base, an aqueous or alcoholic solution of an acid, a solution of a protic organic acid and an inorganic acid, or by hydrogenolysis with a palladium or platinum catalyst; and
adding the deprotected compound of formula 5 to a compound of formula 3 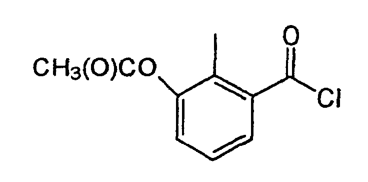 in an organic solvent to form the compound of formula 4,
wherein the acetal-based protecting group is removed under aqueous or alcoholic conditions, promoted by Lewis acids, such as transition metal halides or halides of the group 3 metals, or by protic organic acids, such as p-toluenesulfonic and related alkyl and arylsulfonic acids, trifluoroacetic acid and related organic carboxylic acids with a pK of less than 2, and inorganic acids, such as sulfuric, hydrochloric, phosphoric and nitric acids.

4. A method of making a compound of formula 4 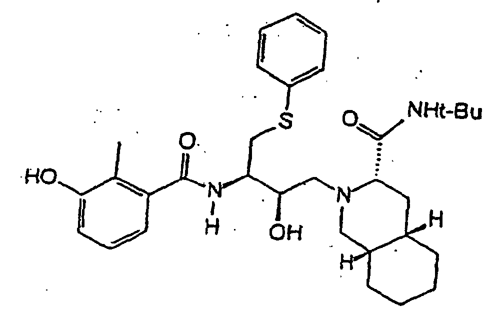 . comprising combining a compound of formula 3 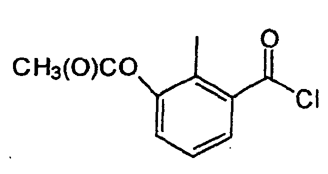 in an organic solvent with a compound of formula 6 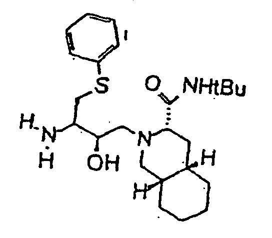 to obtain the compound of formula 4,
wherein the acetal-based protecting group is removed under aqueous or alcoholic conditions, promoted by Lewis acids, such as transition metal halides or halides of the group 3 metals, or by protic organic acids, such as p-toluenesulfonic and related alkyl and arylsulfonic acids, trifluoroacetic acid and related organic carboxylic acids with a pK of less than 2, and inorganic acids, such as sulfuric, hydrochloric, phosphoric and nitric acids.

5. A method of making a compound of formula 7 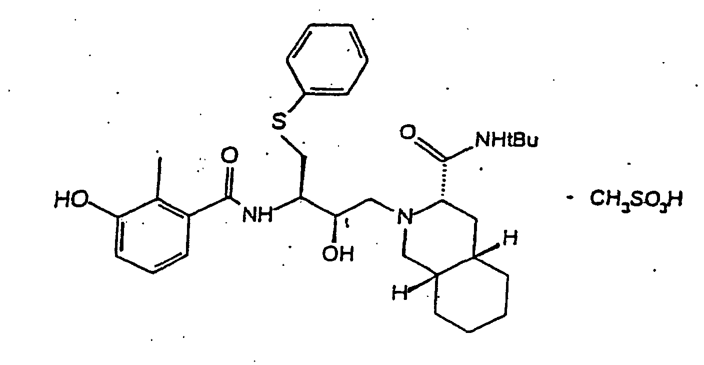 comprising deprotecting a compound of formula 5 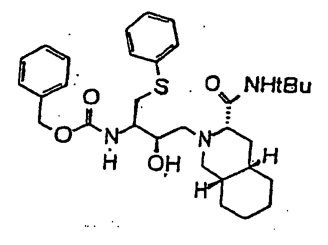 by treatment with an aqueous or alcoholic solution of an inorganic base, an aqueous or alcoholic solution of an acid, a solution of a protic organic acid and an inorganic acid, or by hydrogenolysis with a palladium or platinum catalyst; and
adding the deprotected compound of formula 5 to a compound of formula 3 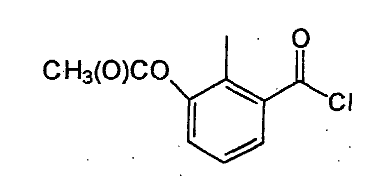 in an organic solvent to form a compound of formula 4, 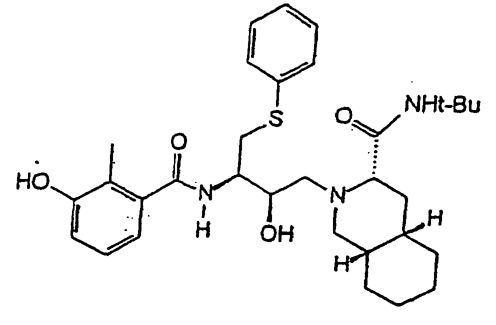 and converting the compound of formula 4 to the compound of formula 7 by treatment with methanesulfonic acid in an organic solvent,
wherein the acetal-based protecting group is removed under aqueous or alcoholic conditions, promoted by Lewis acids, such as transition metal halides or halides of the group 3 metals, or by protic organic acids, such as p-toluenesulfonic and related alkyl and arylsulfonic acids, trifluoroacetic acid and related organic carboxylic acids with a pK of less than 2, and inorganic acids, such as sulfuric, hydrochloric, phosphoric and nitric acids.

6. A method of making a compound of formula 7 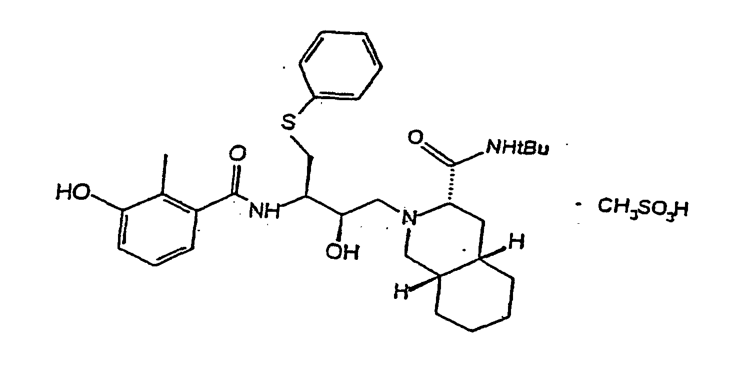 comprising combining a compound of formula 3 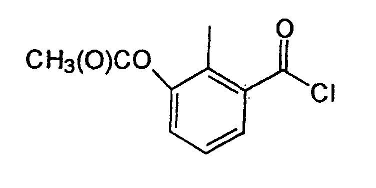 in an organic solvent with a compound of formula 6 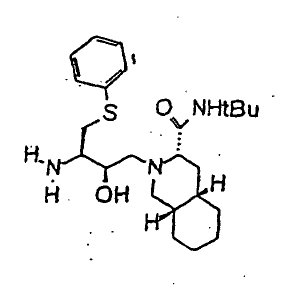 to form a compound of formula 4 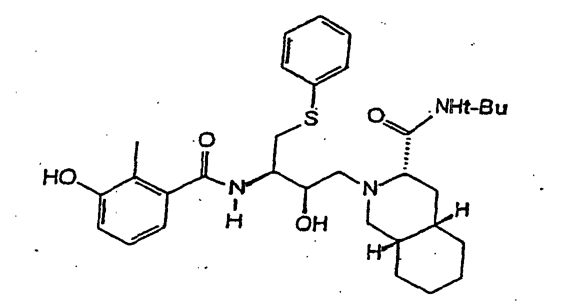 and converting the compound of formula 4 to the compound of formula 7 by treatment with methanesulfonic acid in an organic solvent,
wherein the acetal-based protecting group is removed under aqueous or alcoholic conditions, promoted by Lewis acids, such as transition metal halides or halides of the group 3 metals, or by protic organic acids, such as p-toluenesulfonic and related alkyl and arylsulfonic acids, trifluoroacetic acid and related organic carboxylic acids with a pK of less than 2, and inorganic acids, such as sulfuric, hydrochloric, phosphoric and nitric acids.

## Patentansprüche

1. Verfahren zum Herstellen einer Verbindung der Formel 3 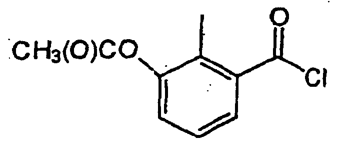 umfassend das Hinzufügen einer Acetylgruppe und einer Abgangsgruppe, bei der es sich um Chlor handelt, zu einer Verbindung der Formel 1, 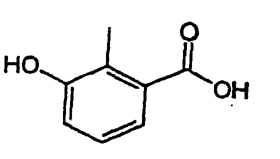 um die Verbindung der Formel 3 zu bilden.

2. Verfahren zum Herstellen einer Verbindung der Formel 3 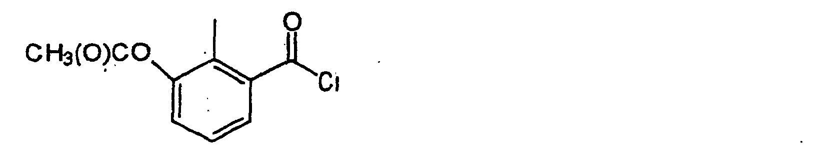 umfassend das Hinzufügen einer Abgangsgruppe, bei der es sich um Chlor handelt, zu einer Verbindung der Formel 2, 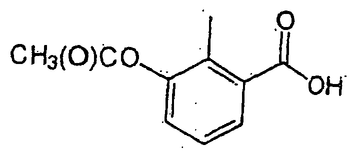 um die Verbindung der Formel 3 zu bilden.

3. Verfahren zum Herstellen einer Verbindung der Formel 4 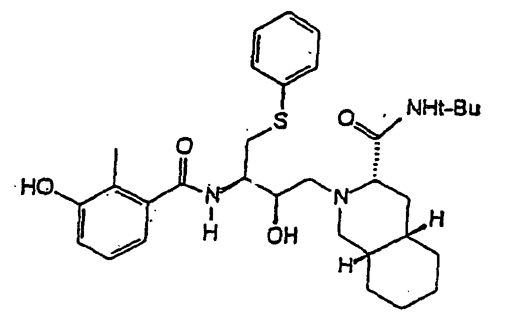 umfassend das Entfernen der Schutzgruppe von einer Verbindung der Formel 5 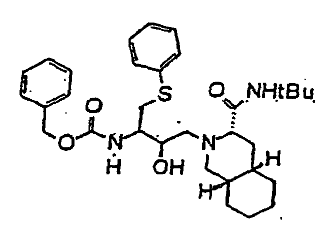 durch Behandlung mit einer wässrigen oder alkoholischen Lösung einer anorganischen Base, einer wässrigen oder alkoholischen Lösung einer Säure, einer Lösung einer protischen organischen Säure und einer anorganischen Säure oder durch Hydrogenolyse mit einem Palladium- oder Platinkatalysator; und das Hinzufügen der Verbindung der Formel 5, von welcher die Schutzgruppe entfernt wurde, zu einer Verbindung der Formel 3 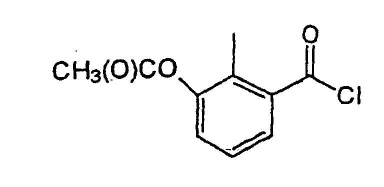 in einem organischen Lösungsmittel, um die Verbindung der Formel 4 zu bilden, wobei die Schutzgruppe auf Acetalbasis unter wässrigen oder alkoholischen Bedingungen, gefördert durch Lewis-Säuren, wie etwa Übergangsmetallhalogenide oder Halogenide von Metallen der Gruppe 3, oder durch protische organische Säuren, wie etwa p-Toluolsulfonsäure und verwandte Alkyl- und Arylsulfonsäuren, Trifluoressigsäure und verwandte organische Carbonsäuren mit einem pK von weniger als 2, und anorganische Säuren, wie etwa Schwefelsäure, Chlorwasserstoffsäure, Phosphorsäure und Salpetersäure, entfernt wird.

4. Verfahren zum Herstellen einer Verbindung der Formel 4 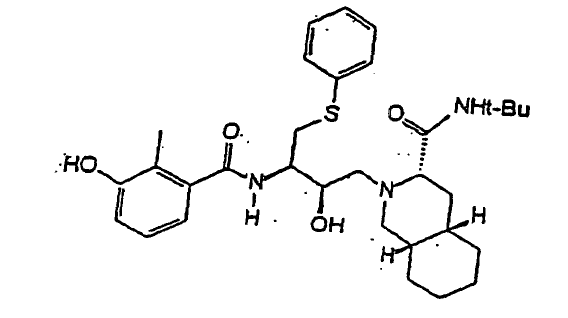 umfassend das Vereinigen einer Verbindung der Formel 3 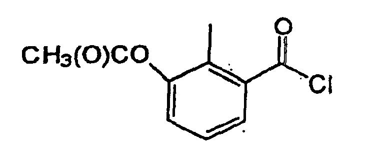 in einem organischen Lösungsmittel mit einer Verbindung der Formel 6 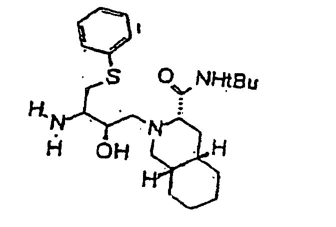 um die Verbindung der Formel 4 zu erhalten,
wobei die Schutzgruppe auf Acetalbasis unter wässrigen oder alkoholischen Bedingungen, gefördert durch Lewis-Säuren, wie etwa Übergangsmetallhalogenide oder Halogenide von Metallen der Gruppe 3, oder durch protische organische Säuren, wie etwa p-Toluolsulfonsäure und verwandte Alkyl- und Arylsulfonsäuren, Trifluoressigsäure und verwandte organische Carbonsäuren mit einem pK von weniger als 2, und anorganische Säuren, wie etwa Schwefelsäure, Chlorwasserstoffsäure, Phosphbrsäure und Salpetersäure, entfernt wird.

5. Verfahren zum Herstellen einer Verbindung der Formel 7 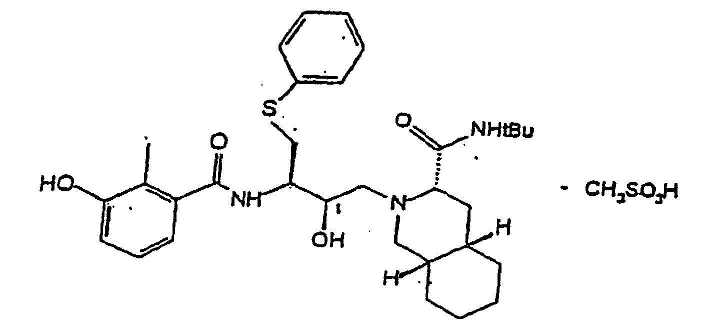 umfassend das Entfernen der Schutzgruppe von einer Verbindung der Formel 5 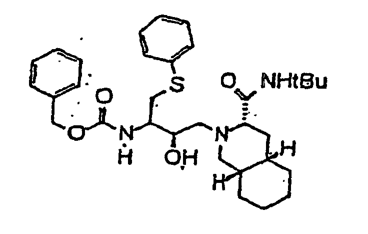 durch Behandlung mit einer wässrigen oder alkoholischen Lösung einer anorganischen Base, einer wässrigen oder alkoholischen Lösung einer Säure, einer Lösung einer protischen organischen Säure und einer anorganischen Säure oder durch Hydrogenolyse mit einem Palladium- oder Platinkatalysator; und Hinzufügen der Verbindung der Formel 5, von der die Schutzgruppe entfernt wurde, zu einer Verbindung der Formel 3 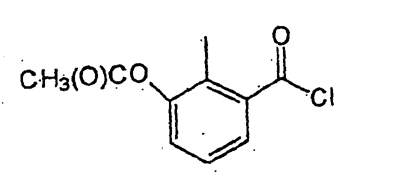 in einem organischen Lösungsmittel, um eine Verbindung der Formel 4 zu bilden, 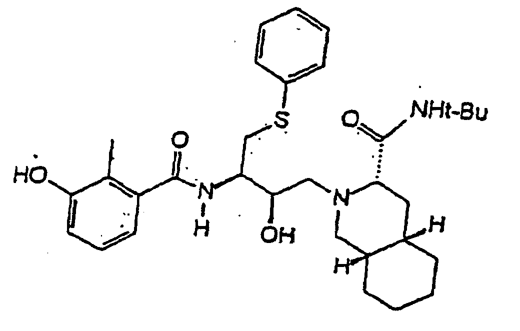 und das Umwandeln der Verbindung der Formel 4 in die Verbindung der Formel 7 durch Behandlung mit Methansulfonsäure in einem organischen Lösungsmittel, wobei die Schutzgruppe auf Acetalbasis unter wässrigen oder alkoholischen Bedingungen, gefördert durch Lewis-Säuren, wie etwa Übergangsmetallhalogenide oder Halogenide von Metallen der Gruppe 3, oder durch protische organische. Säuren, wie etwa p-Toluotsulfonsäure und verwandte Alkyl- und Arylsulfonsäuren, Trifluoressigsäure und verwandte organische Carbonsäuren mit einem pK von weniger als 2, und anorganische Säuren, wie etwa Schwefelsäure, Chlorwasserstoffsäure, Phosphorsäure und Salpetersäure, entfernt wird.

6. Verfahren zum Herstellen einer Verbindung der Formel 7 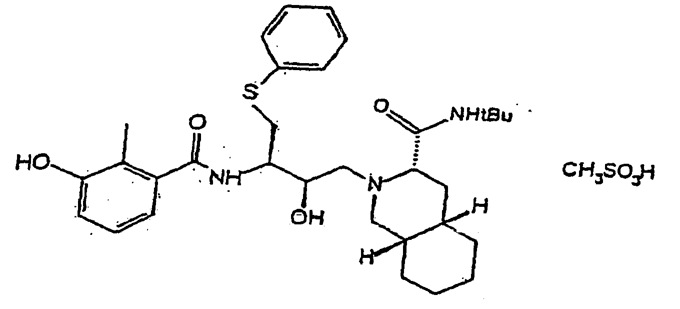 umfassend das Vereinigen einer Verbindung der Formel 3 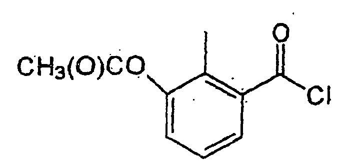 in einem organischen Lösungsmittel mit einer Verbindung der Formel 6 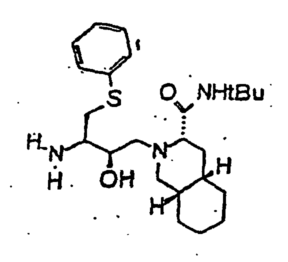 um eine Verbindung der Formel 4 zu bilden 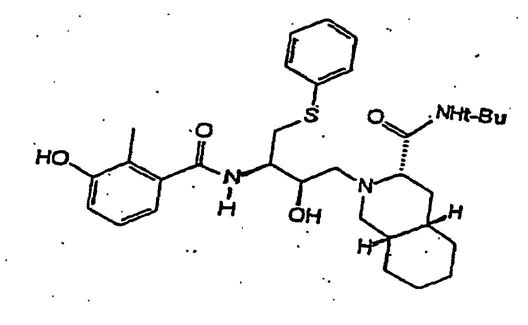 und das Umwandeln der Verbindung der Formel 4 in die Verbindung der Formel 7 durch Behandlung mit Methansulfonsäure in einem organischen Lösungsmittel, wobei die Schutzgruppe auf Acetalbasis unter wässrigen oder alkoholischen Bedingungen, gefördert durch Lewis-Säuren, wie etwa Übergangsmetallhalogenide oder Halogenide von Metallen der Gruppe 3, oder durch protische organische Säuren, wie etwa p-Toluolsulfonsäure und verwandte Alkyl- und Arylsulfonsäuren, Trifluoressigsäure und verwandte organische Carbonsäuren mit einem pK von weniger als 2, und anorganische Säuren, wie etwa Schwefelsäure, Chlorwasserstoffsäure, Phosphorsäure und Salpetersäure, entfernt wird.

## Revendications

1. Procédé permettant de préparer un composé de formule 3 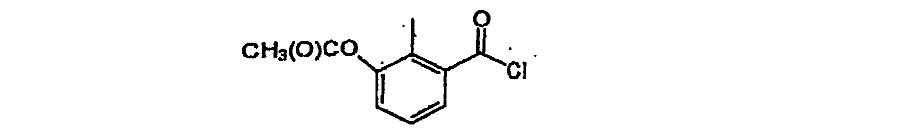 comprenant l'ajout d'un groupe acétyle et d'un groupe partant qui est du chlore à un composé de formule 1 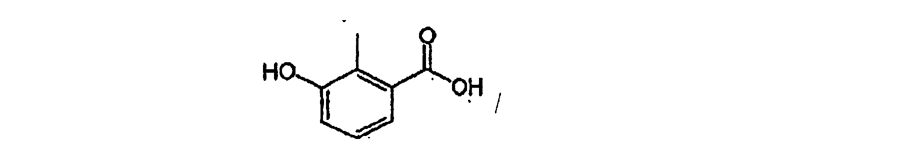 afin de former le composé de formule 3.

2. Procédé permettant de préparer un composé de formule 3 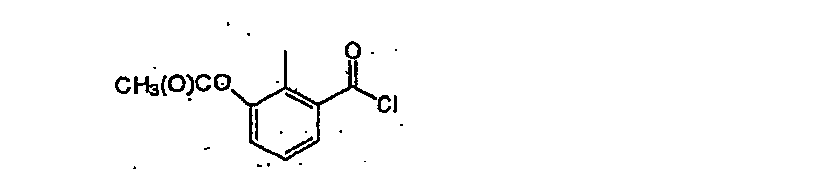 comprenant l'ajout d'un groupe partant lequel est du chlore à un composé de formule 2 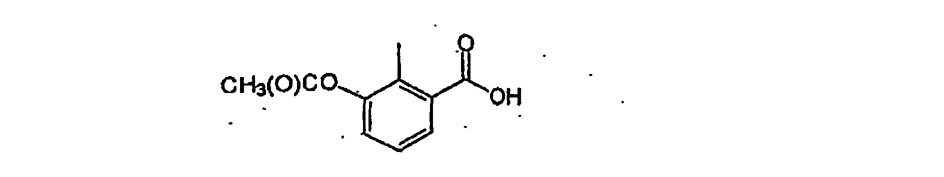 afin de former le composé de formule 3.

3. procédé permettant de préparer un composé de formule 4 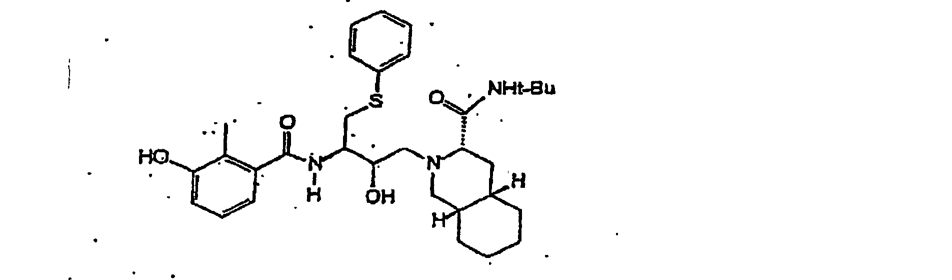 comprenant la déprotection d'un composé de formule 5 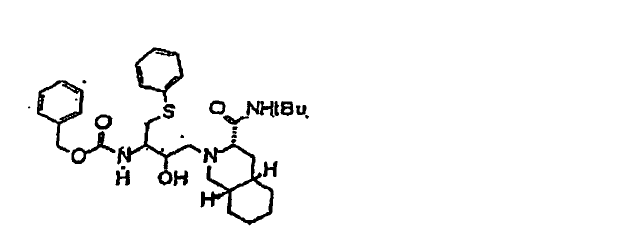 au moyen d'un traitement avec une solution aqueuse ou alcoolique d'une base inorganique, une solution aqueuse ou alcoolique d'un acide, une solution d'un acide organique protique et d'un acide inorganique ou au moyen d'une hydrogénolyse à l'aide d'un catalyseur au palladium ou au platine ; et l'ajout du composé déprotégé de formule 5 au composé de formule 3 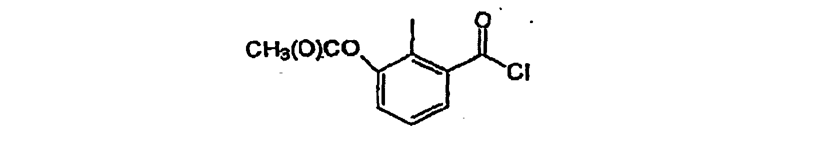 dans un solvant organique afin d'obtenir le composé de formule 4, dans lequel le groupe protecteur à base d'acétal est supprimé sous des conditions aqueuses ou alcooliques, promues par les acides de Lewis, tels que les halogénures de métal de transition ou les halogénures des métaux du groupe 3, ou par les acides organiques protiques, tels que les acides p-toluènes sulfoniques et ses acides alkyle et arylsulfoniques qui s'y rapportent, l'acide trifloroacétique et les acides carboxyliques organiques qui s'y rapportent présentant un pK inférieur à 2, et les acides inorganiques tels que les acides sulfuriques, chlorhydriques, phosphoriques et nitriques.

4. Procédé permettant de préparer un composé de formule 4 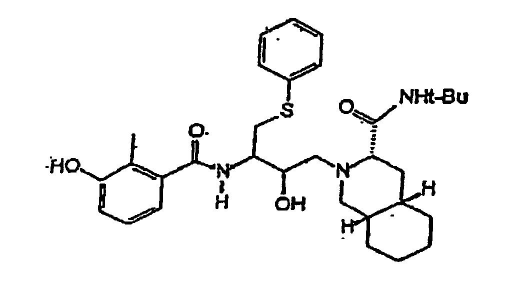 comprenant la combinaison d'un composé de formule 3 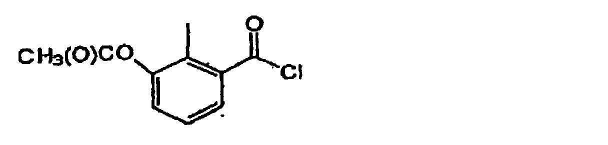 dans un solvant organique avec un composé de formule 6 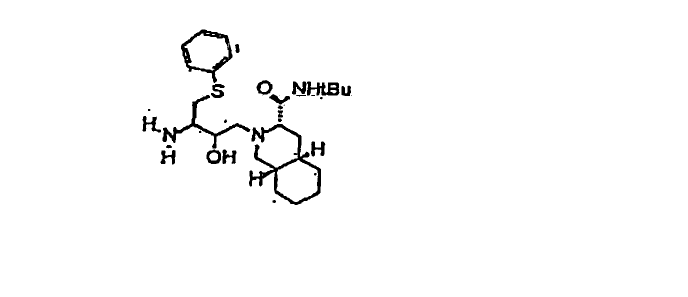 afin d'obtenir le composé de formule 4,
dans lequel le groupe protecteur à base d'acétal est supprimé sous des conditions aqueuses ou alcooliques, promues par les acides de Lewis, tels que les halogénures de métal de transition ou des halogénures des métaux du groupe 3, ou par les acides organiques protiques, tels que les acides p-toluènes sulfoniques et ses acides alkyle et arylsulfoniques qui s'y rapportent, l'acide trifloroacétique et les acides carboxyliques organiques qui s'y rapportent présentant un pK inférieur à 2, et les acides inorganiques tels que les acides sulfuriques, chlorhydriques, phosphoriques et nitriques.

5. Procédé permettant de préparer un composé de formule 7 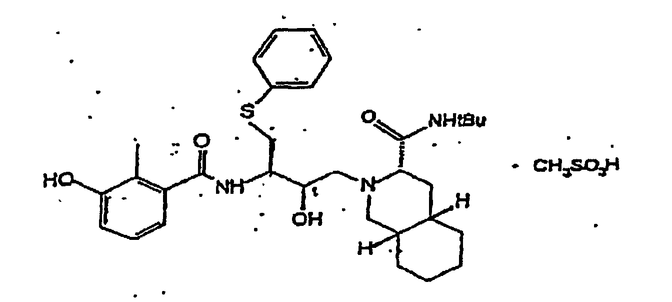 comprenant la déprotection d'un composé de formule 5 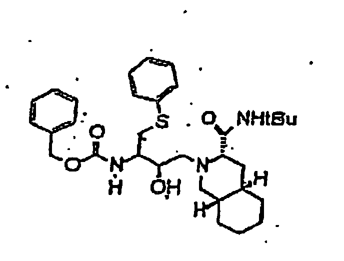 au moyen d'un traitement à l'aide d'une solution aqueuse ou alcoolique d'une base inorganique, une solution aqueuse ou alcoolique d'un acide, une solution d'un acide organique protique et d'un acide inorganique, ou au moyen d'une hydrogénolyse à l'aide d'un catalyseur au palladium ou au platine ; et l'ajout du composé déprotégé de formule 5 au composé de formule 3 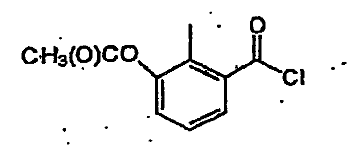 dans un solvant organique afin d'obtenir le composé de formule 4 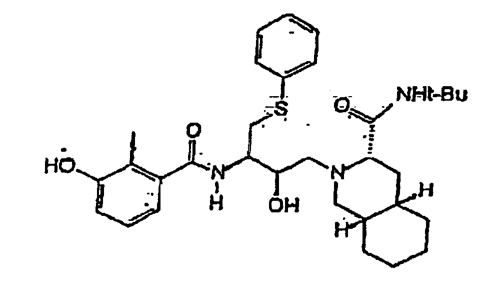 et la conversion du composé de formule 4 en composé de formule 7 au moyen d'un traitement à l'aide d'acide méthane sulfonique dans un solvant organique, dans lequel le groupe protecteur à base d'acétal est supprimé sous des conditions aqueuses ou alcooliques, promues par les acides de Lewis, tels que les halogénures de métal de transition ou des halogénures des métaux du groupe 3, ou par les acides organiques protiques, tels que les acides p-toluène sulfoniques et des acides alkyle et arylsulfoniques qui s'y rapportent, l'acide trifloroacétique et les acides carboxyliques organiques qui s'y rapportent présentant un pK inférieur à 2, et les acides inorganiques tels que les acides sulfuriques, chlorhydriques, phosphoriques et nitriques.

6. Procédé permettant de préparer un composé de formule 7 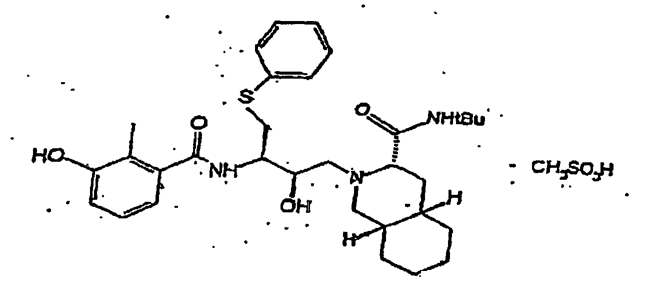 comprenant la combinaison d'un composé de formule 3 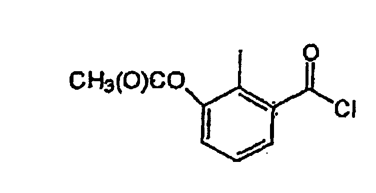 dans un solvant organique avec un composé de formule 6 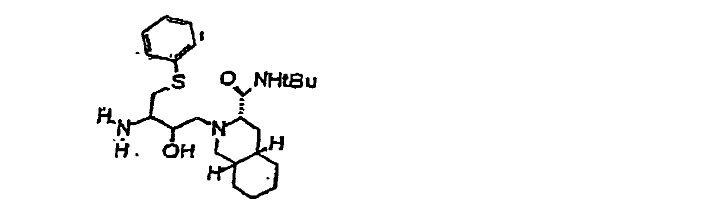 afin d'obtenir un composé de formule 4 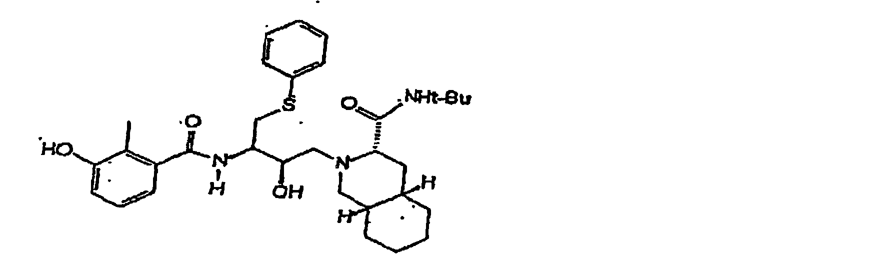 et la conversion du composé de formule 4 en composé de formule 7 au moyen d'un traitement à l'aide d'acide méthane sulfonique dans un solvant organique, dans lequel le groupe protecteur à base d'acétal est supprimé sous des conditions aqueuses ou alcooliques, promues par les acides de Lewis, tels que les halogénures de métal de transition ou des halogénures des métaux du groupe 3, ou par les acides organiques protiques, tels que les acides p-toluènes sulfoniques et les acides alkyle et arylsulfoniques qui s'y rapportent, l'acide trifloroacétique et les acides carboxyliques organiques qui s'y rapportent présentant un pK inférieur à 2, et les acides inorganiques tels que les acides sulfuriques, chlorhydriques, phosphoriques et nitriques.
